# EUROPEAN PATENT APPLICATION

(11) **EP 3 156 406 A1**
(43) Date of publication of application: **19.04.2017**
(21) Application number: 15189697.4
(22) Date of filing: 14.10.2015
(51) Int. Cl.: C07D 487/04, A61K 31/519, A61P 29/00, A61P 35/00, A61P 37/00

(54) **CRYSTALLINE FORMS OF RIBOCICLIB FREE BASE**

(71) Applicant: ratiopharm GmbH, 89079 Ulm (DE)
(72) Inventor: ALBRECHT, Wolfgang, 89075 Ulm (DE); LEHMANN, Frank, 89233 Neu-Ulm (DE)
(74) Representative: Teipel, Stephan

(57) **Abstract**

The present invention relates to crystalline ribociclib free base, methods of its preparation and a pharmaceutical composition comprising the same.

## Description

### Background of the invention

The present invention relates to crystalline ribociclib free base, methods of preparing the same as well as a pharmaceutical composition comprising the same.

The IUPAC name of ribociclib is 7-cyclopentyl-N,N-dimethyl-2-((5-(piperazin-1-yl)pyridin-2-yl)amino)-7H-pyrrolo[2,3-d]pyrimidine-6-carboxamide. Ribociclib is represented by the following chemical structure according to Formula (I):

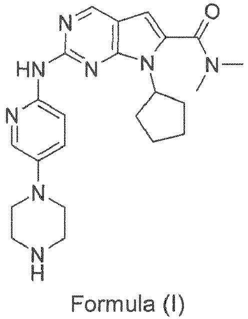

Ribociclib is an orally available cyclin-dependent kinase (CDK) inhibitor targeting cyclin D1/CDK4 and cyclin D3/CDK6 cell cycle pathway, with potential antineoplastic activity. Ribociclib specifically inhibits CDK4 and 6, thereby inhibiting retinoblastoma (Rb) protein phosphorylation. Inhibition of Rb phosphorylation prevents CDK-mediated G1-S phase transition, thereby arresting the cell cycle in the G1 phase, suppressing DNA synthesis and inhibiting cancer cell growth. Overexpression of CDK4/6, as seen in certain types of cancer, causes cell cycle deregulation.

The active pharmaceutical ingredient ribociclib is known from WO 2010/020675. No details on the solid state of the final form are disclosed in this document.

WO 2012/064805 discloses a new process for preparation of ribociclib and the succinate salt thereof. In addition, this application also describes the solid state characteristics of anhydrous ribociclib succinate salt as well as the monohydrate thereof.

However, when following the proposed route of synthesis the obtained ribociclib shows certain disadvantages, e.g. with regard to its purity and/or solid state.

For example, the conversion of one polymorphic form into another polymorphic form can be unfavourable in pharmaceutical dosage forms such as tablets, often resulting in different hygroscopicity, dissolution and pharmacokinetic properties. As a result thereof, the bioavailability of the active agent might be undesirably unpredictable. Consequently, active agents having different interchangeable polymorphs may lead to regulatory and commercial disadvantages since they very often do not fulfil the requirements of the corresponding regulation authorities such as the FDA and EMEA.

Further, it was found that ribociclib crystallizes in several different solid forms, i.e. polymorphic crystalline and amorphous forms. However, due to the preparation methods several ribociclib solid forms contain an unacceptably high residual solvent content. Such residual solvents have to be removed nearly completely from potential pharmaceutical formulations, or at least to a level that is acceptable by the FDA (Food and Drug Association in the US). According to FDA regulations, solvents like dichloromethane, dioxane and ethanol are only allowable in amounts of 600 ppm, 380 ppm and 5000 ppm, respectively. These values are difficult to achieve since ribociclib obtained in known preparation processes often shows a higher content of residual solvents and employing conventional drying methods like drying at elevated temperatures with and without vacuum does not lead to pharmaceutically acceptable solvent levels and/or drug purity levels.

Consequently, there is still a need for stable solid forms of ribociclib having a pharmaceutically acceptable purity, desired solubility and/or hygroscopicity.

Hence, it was an object of the present invention to overcome the drawbacks of the above-mentioned prior art.

Additionally ribociclib should be provided in a form which is easy to handle and/or stable over a long period.

### Summary of the invention

According to the present invention, the above objectives are unexpectedly achieved by providing crystalline ribociclib in form of its free base having a residual solvent content within pharmaceutically acceptable limits, methods for preparing the same and a pharmaceutical composition comprising such crystalline ribociclib free base.

Thus, the subject of the present invention is crystalline ribociclib free base having a residual solvent content within pharmaceutically acceptable limits.

### Detailed description of the invention

In the present application the term "residual solvent content within pharmaceutically acceptable limits" refers to a concentration limit (in ppm) of the corresponding solvent, which is regarded as pharmaceutically acceptable in the art.

Respective concentration limits for solvents can be found in Guidance for Industry, Q3C-Tables and List, U.S. Department of Health and Human Services, Food and Drug Administration, Center for Drug Evaluation and Research (CDER), Center for Bioligics Evaluation and Research (CBER), February 2012, ICH, Revision 2.

According to the above-mentioned guideline the solvents are categorized into three classes.

Class 1 relates to solvents that should be avoided in pharmaceutical products.

**Table 1: Class 1 solvents and their concentration limits**

| **Solvent** | **Concentration Limit (ppm)** | **Concern** |
|---|---|---|
| Benzene | 2 | Carcinogen |
| Carbon tetrachloride | 4 | Toxic and environmental hazard |
| 1,2-Dichloroethane | 5 | Toxic |
| 1,1-Dichloroethane | 8 | Toxic |
| 1,1,1-Trichloroethane | 1,500 | Environmental hazard |

Class 2 relates to solvents that should be limited in pharmaceutical products because of their inherent toxicity.

**Table 2: Class 2 solvents and their concentration limits**

| **Solvent** | **PDE (mg/day)** | **Concentration Limit (ppm)** |
|---|---|---|
| Acetonitrile | 4.1 | 410 |
| Chlorobenzene | 3.6 | 360 |
| Chloroform | 0.6 | 60 |
| Cyclohexane | 38.8 | 3,880 |
| Cumene | 0.7 | 70 |
| 1,2-Dichloroethane | 18.7 | 1,870 |
| Dichloromethane | 6.0 | 600 |
| 1,2-Dimethoxyethane | 1.0 | 100 |
| N,N-Dimethylacetamide | 10.9 | 1,090 |
| N, N-Dimethylformamide | 8.8 | 880 |
| 1,4-Dioxane | 3.8 | 380 |
| 2-Ethoxyethanol | 1.6 | 160 |
| Ethyleneglycol | 6.2 | 620 |
| Formamide | 2.2 | 220 |
| Hexane | 2.9 | 290 |
| Methanol | 30.0 | 3,000 |
| 2-Methoxyethanol | 0.5 | 50 |
| Methylbutyl ketone | 0.5 | 50 |
| Methylcyclohexane | 11.8 | 1,180 |
| N-Methylpyrrolidone | 5.3 | 530 |
| Nitromethane | 0.5 | 50 |
| Pyridine | 2.0 | 200 |
| Sulfolane | 1.6 | 160 |
| Tetrahydrofuran | 7.2 | 720 |
| Tetralin | 1.0 | 100 |
| Toluene | 8.9 | 890 |
| 1,1,1-Trichloroethene | 0.8 | 80 |
| Xylene¹ | 21.7 | 2,170 |

| | | |
|---|---|---|
| ¹Usually 60% m-xylene, 14% p-xylene, 9% o-xylene with 17% ethyl benzene | | |

Class 3 relates to solvents which should be limited by GMP or other quality-based requirements. These solvents should be present in an amount below 5000 ppm.

**Table 3: Class 3 solvents having a concentration limit of 5000 ppm.**

| | |
|---|---|
| Acetic acid | Heptane |
| Acetone | Isobutyl acetate |
| Anisole | Isopropyl acetate |
| 1-Butanol | Methyl acetate |
| 2-Butanol | 3-Methyl-1-butanol |
| Butyl acetate | Methylethyl ketone |
| *tert*-Butylmethyl ether | Methylisobutyl ketone |
| Dimethyl sulfoxide | 2-Methyl-1-propanol |
| Ethanol | Pentane |
| Ethyl acetate | 1-Pentanol |
| Ethyl ether | 1-Propanol |
| Ethyl formate | 2-Propanol |
| Formic acid | Propyl acetate |

The term "crystalline" can be used in the context of this invention to designate the state of solid substances in which the components (atoms, ions or molecules, i.e. in the case of crystalline ribociclib the ribociclib molecules) are arranged in an orderly repeating pattern, extending in all three spatial dimensions and thus exhibit a periodic arrangement over a great range (= long-range order).

In a preferred embodiment the crystalline ribociclib may consist of purely crystalline ribociclib. Alternatively, it may also contain small amounts of non-crystalline ribociclib components, provided that a defined melting point of crystalline ribociclib can be detected in DSC. Preferably, the crystalline ribociclib contains a maximum of 15% by weight non-crystalline ribociclib, more preferably a maximum of 10% by weight, even more preferably a maximum of 5% by weight, such as a maximum of 1 % by weight, each based on the total weight of the ribociclib free base. It is preferred that ribociclib according to the invention can be a mixture containing 85 to 99.999% by weight crystalline ribociclib and 0.001 to 15% by weight non-crystalline ribociclib, more preferably 90 to 99.99% by weight crystalline ribociclib and 0.01 to 10% by weight non-crystalline ribociclib, particularly preferably 95 to 99.9% by weight crystalline ribociclib and 0.1 to 5% by weight non-crystalline ribociclib.

Crystalline ribociclib free base can be present in different crystal forms. Thus, crystalline ribociclib can be present in different polymorphic forms or mixtures thereof.

A crystal form may be referred to herein as being characterized by data selected from two or more different data groupings, for example by a powder XRD pattern having a group of specific peaks or by a powder XRD pattern as shown in a figure depicting a diffractogram or by "a combination thereof' (or "combinations thereof" or "any combination thereof'). These expressions, e.g. "any combination thereof', contemplate that the skilled person may characterize a crystal form using any combination of the recited characteristic analytical data. For example, the skilled person may characterize a crystal form using a group of three, four or five characteristic powder XRD peaks and supplement that characterization with one or more additional features observed in the powder X-ray diffractogram, e.g., an additional peak, a characteristic peak shape, a peak intensity or even the absence of a peak at some position in the powder XRD pattern. Alternatively, the skilled person may in some instances characterize a crystal form using a group of three, four or five characteristic powder XRD peaks and supplement that characterization with one or more additional feature(s) observed using another analytical method, for example using one or more characteristic peaks in a solid state IR spectrum, solid state NMR or characteristics of the DSC thermogram of the crystal form that is being characterized.

Unless indicated otherwise, XRPD peaks are recorded using copper Kα₁/ Kα₂ radiation with a wavelength 1.5406 A (weighted mean of Cu Kα₁ and Cu Kα₂). Further, unless indicated otherwise, XRPD peaks are reported as degrees 2θ (2-theta) values with a standard error of ± 0.2 degrees 2θ (2-theta).

A crystal form may be referred to herein as being characterized by graphical data "as depicted in" a particular figure. Such data include for example powder X-ray diffractograms. The skilled person will understand that such graphical representations of data may be subject to small variations, e.g. in peak relative intensities and peak positions due to factors such as variations in instrument response and variations in sample concentration and purity, which are well known to the skilled person. Nonetheless, the skilled person would readily be capable of comparing the graphical data in the figures herein with graphical data generated for an unknown crystal form and confirm whether the two sets of graphical data are characterizing the same crystal form or two different crystal forms.

Ribociclib in form of the free base can be illustrated by Formula (I). This means that the free base form does not encompass a salt form. Thus, ribociclib as free base form preferably corresponds to ribociclib free base. Further, ribociclib free base can encompass ribociclib in form of hydrate and/or solvate.

According to a first embodiment of the invention crystalline ribociclib free base relates to the crystal Form 1 of ribociclib free base. This form can be considered as polymorphic Form 1 of ribociclib. Ribociclib free base Form 1 is preferably characterized by XRPD peaks at 12.5, 12.6, 13.3, 14.8 and 18.3 degrees 2θ (± 0.2 degrees 2θ).

In a preferred embodiment ribociclib free base Form 1 is characterized by one or more further XRPD peak(s) at 13.6, 18.1, 21.8, 22.9 and/or 26.9 degrees 2θ (± 0.2 degrees 2θ).

In a further preferred embodiment ribociclib free base Form 1 is characterized by the following XRPD peaks at degrees 2θ ± 0.2 degrees 2θ: 6.8, 8.1, 10.0, 12.5, 12.6, 13.3, 13.6, 14.8, 15.2, 15.7, 16.4, 18.1, 18.3, 18.5, 20.1, 20.4, 21.4, 21.8, 22.6, 22.9, 23.4, 23.8, 24.3, 24.6, 26.2, 26.9, 27.2, 29.2, 29.8, 31.7, 33.7, 34.4.

In a further preferred embodiment ribociclib free base Form 1 is characterized by the following XRPD peaks at degrees 2θ ± 0.2 degrees 2θ (Intensity %): 6.8 (13.4%), 8.1 (10.1%), 10.0 (3.0%), 12.5 (47.1%), 12.6 (50.2%), 13.3 (28.7%), 13.6 (16.2%), 14.8 (24.6%), 15.2 (10.6%), 15.7 (14.2%), 16.4 (7.0%), 18.1 (92.2%), 18.3 (100.0%), 18.5 (56.2%), 20.1 (59.7%), 20.4 (90.6%), 21.4 (28.4%), 21.8 (42.7%), 22.6 (45.6%), 22.9 (48.9%), 23.4 (14.8%), 23.8 (19.4%), 24.3 (8.4%), 24.6 (12.9%), 26.2 (18.2%), 26.9 (40.2%), 27.2 (8.6%), 29.2 (17.4%), 29.8 (7.9%), 31.7 (15.1%), 33.7 (7.9%), 34.4 (8.9%).

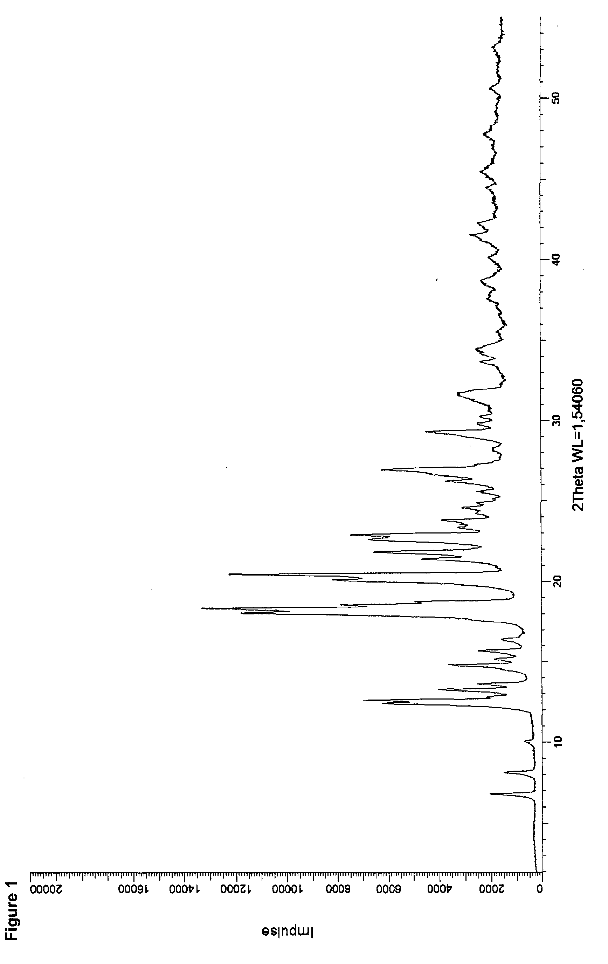

An XRPD pattern of ribociclib free base Form 1 is shown in Figure 1.

Due to the easy access and/or the good solubility in organic solvents ribociclib free base Form 1 can advantageously be used to prepare pharmaceutically acceptable acid addition salts of ribociclib.

Crystalline ribociclib free base Form 1 can be prepared by slurrying amorphous ribociclib free base in acetone, for example for 12 to 48 hours. Preferably for about 24 hours. Slurrying can be conducted for example at 10 to 35°C, preferably at room temperature (RT), such as about 23°C.

It is further preferred that the slurry is subjected to a mechanical movement such as stirring.

The thus obtained mixture can be filtrated and the obtained solid can be dried. The step of drying can preferably be carried out at a temperature of 30°C to 70°C, preferably of 40°C to 60°C, such as about 50°C. Alternatively or additionally the drying can be carried out under reduced pressure of from 1 to 500 mbar, in particular 10 to 20 mbar.

According to a second embodiment of the invention crystalline ribociclib free base relates to the crystal **Form 6** of ribociclib free base. This form can be considered as polymorphic Form 6 of ribociclib. Ribociclib free base Form 6 is preferably characterized by XRPD peaks at 6.4, 11.3, 16.6, 19.1 and 22.4 degrees 2θ (± 0.2 degrees 2θ).

In a preferred embodiment ribociclib free base Form 6 is characterized by one or more further XRPD peak(s) at 8.2, 15.3, 17.7, 20.2 and/or 28.8 degrees 2θ (± 0.2 degrees 2θ).

In a further preferred embodiment ribociclib free base Form 6 is characterized by the following XRPD peaks at degrees 2θ ± 0.2 degrees 2θ (Intensity %): 6.4, 8.2, 9.4, 11.3, 12.4, 13.0, 14.1, 15.3, 16.6, 16.8, 17.7, 19.1, 20.2, 22.4, 23.3, 24.9, 26.0, 26.9, 28.8, 30.8, 33.0, 33.4.

In a further preferred embodiment ribociclib free base Form 6 is characterized by the following XRPD peaks at degrees 2θ ± 0.2 degrees 2θ (Intensity %): 6.4 (11.2%), 8.2 (7.6%), 9.4 (4.4%), 11.3 (77.5%), 12.4 (2.4%), 13.0 (3.2%), 14.1 (1.9%), 15.3 (16.9%), 16.6 (38.5%), 16.8 (20.4%), 17.7 (21.7%), 19.1 (84.5%), 20.2 (47.1%), 22.4 (100.0%), 23.3 (25.7%), 24.9 (14.8%), 26.0 (9.9%), 26.9 (24.8%), 28.8 (19.3%), 30.8 (18.8%), 33.0 (13.9%), 33.4 (11.8%).

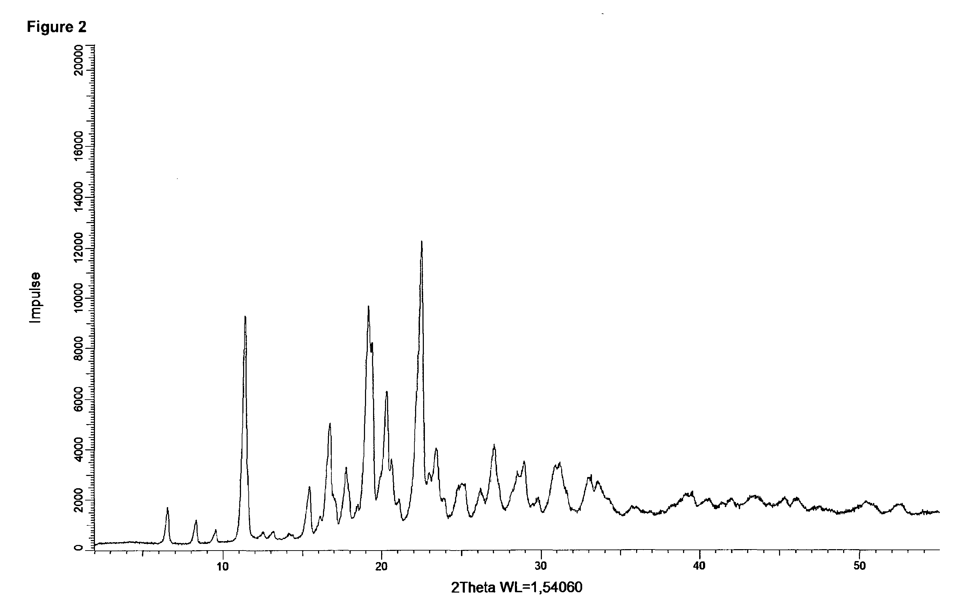

An XRPD pattern of ribociclib free base Form 6 is shown in Figure 2.

Due to the easy access and/or the good solubility in organic solvents ribociclib free base Form 6 can advantageously be used to prepare pharmaceutically acceptable acid addition salts of ribociclib.

Crystalline ribociclib free base Form 6 can be prepared by slurrying ribociclib free base of any form being different from Form 6 in water or dissolving ribociclib free base in an aqueous solvent, such as water, at low pH, preferably at a pH below 3, more preferably below 2, and precipitating crystalline ribociclib free base from the obtained solution by increasing the pH, preferably above 10, more preferably above 11.

Slurrying can be conducted for 12 to 48 hours, preferably for about 24 hours. Furthermore, slurrying can be conducted for example at 10 to 35°C, preferably at room temperature, such as about 23°C.

It is further preferred that the slurry is subjected to a mechanical movement such as stirring.

Dissolving ribociclib free base in an aqueous solvent, such as aqueous HCl, can be conducted at a temperature of for example between 10 and 40°C, such as room temperature (about 23°C). Precipitation occurs upon increasing the pH, for example by adding NaOH. Precipitation can be completed by storing the solution at room temperature for 2 to 12 hours, such as for about 6 hours.

The thus obtained mixtures can be filtrated and the obtained solid can be dried. The step of drying can preferably be carried out at a temperature of 30°C to 70°C, preferably of 40°C to 60°C, such as about 50°C. Alternatively or additionally the drying can be carried out under reduced pressure of from 1 to 500 mbar, in particular 10 to 20 mbar.

According to a third embodiment of the invention crystalline ribociclib free base relates to the crystal **Form 7** of ribociclib free base. This form can be considered as polymorphic Form 7 of ribociclib. Ribociclib free base Form 7 is characterized by XRPD peaks at 5.3, 11.0, 13.9, 14.7 and 15.9 degrees 2θ (± 0.2 degrees 2θ).

In a preferred embodiment ribociclib free base Form 7 is characterized by one or more further XRPD peak(s) at 10.7, 13.0, 20.8, 21.1 and/or 23.2 degrees 2θ (± 0.2 degrees 2θ).

In a further preferred embodiment ribociclib free base Form 7 is characterized by the following XRPD peaks at degrees 2θ ± 0.2 degrees 2θ: 5.3, 9.6, 10.0, 10.7, 11.0, 13.0, 13.9, 14.5, 14.7, 15.9, 17.0, 17.4, 17.9, 18.8, 19.1, 19.6, 19.9, 20.8, 21.0, 21.1, 21.4, 22.1, 23.2, 23.8, 24.7, 24.6, 25.2, 25.5, 26.2, 27.5, 34.1.

In a further preferred embodiment ribociclib free base Form 7 is characterized by the following XRPD peaks at degrees 2θ ± 0.2 degrees 2θ (Intensity %): 5.3 (22.4%), 9.6 (4.9%), 10.0 (2.1%), 10.7 (20.5%), 11.0 (74.7%), 13.0 (16.9%), 13.9 (15.6%), 14.5 (13.7%), 14.7 (23.6%), 15.9 (24.8%), 17.0 (1.4%), 17.4 (1.9%), 17.9 (2.3%), 18.8 (16.7%), 19.1 (29.2%), 19.6 (22.7%), 19.9 (35.1%), 20.8 (100.0%), 21.0 (54.5%), 21.1 (65.8%), 21.4 (29.3%), 22.1 (15.3%), 23.2 (44.8%), 23.8 (12.8%), 24.7 (9.1%), 24.6 (16.2%), 25.2 (9.3%), 25.5 (6.1%), 26.2 (16.6%), 27.5 (12.5%), 34.1 (8.9%).

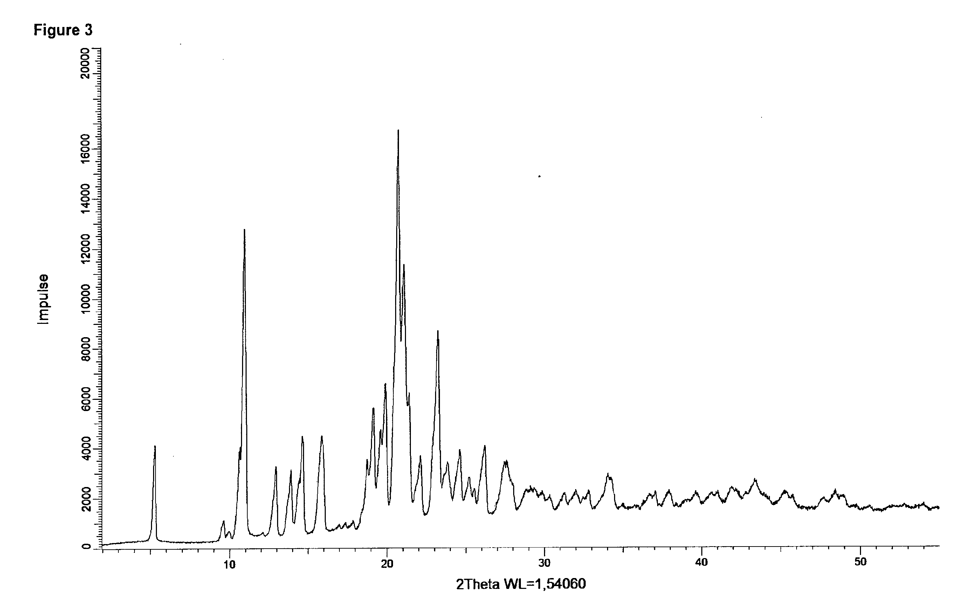

An XRPD pattern of ribociclib free base Form 7 is shown in Figure 3.

Due to the easy access and/or the good solubility in organic solvents ribociclib free base Form 7 can advantageously be used to prepare pharmaceutically acceptable acid addition salts of ribociclib.

Crystalline ribociclib free base Form 7 can be prepared by dissolving ribociclib free base in ethylacetate and precipitating crystalline free base from the obtained solution.

Dissolving can be conducted at elevated temperature of for example 40 to 70°C, preferably 45 to 60°C, such as about 50°C. Crystallization is obtained by slowly cooling the obtained solution to room temperature, in particular to about 23°C.

The thus obtained mixture can be filtrated and the obtained solid can be dried. The step of drying can preferably be carried out at a temperature of 30°C to 70°C, preferably of 40°C to 60°C, such as about 50°C. Alternatively or additionally the drying can preferably be carried out under reduced pressure of from 1 to 500 mbar, in particular 10 to 20 mbar.

According to a fourth embodiment of the invention crystalline ribociclib free base relates to the crystal **Form 8** of ribociclib free base. This form can be considered as polymorphic Form 8 of ribociclib. Ribociclib free base Form 8 is characterized by XRPD peaks at 9.1, 11.1, 15.2, 18.9 and 20.9 degrees 2θ (± 0.2 degrees 2θ).

In a preferred embodiment ribociclib free base Form 8 is characterized by one or more further XRPD peak(s) at 7.6, 8.2, 12.7, 26.7 and/or 28.2 degrees 2θ (± 0.2 degrees 2θ).

In a further preferred embodiment ribociclib free base Form 8 is characterized by the XRPD peaks at degrees 2θ ± 0.2 degrees 2θ (Intensity %): 7.6, 8.2, 9.1, 9.6, 11.1, 12.7, 15.2, 16.4, 16.9, 18.9, 19.3, 20.9, 23.1, 24.0, 25.9, 26.7, 28.2, 31.1, 32.1, 33.3, 42.9.

In a further preferred embodiment ribociclib free base Form 8 is characterized by the XRPD peaks at degrees 2θ ± 0.2 degrees 2θ (Intensity %): 7.6 (3.4%), 8.2 (5.4%), 9.1 (18.0%), 9.6 (7.8%), 11.1 (22.5%), 12.7 (16.7%), 15.2 (34.5%), 16.4 (6.6%), 16.9 (8.7%), 18.9 (56.8%), 19.3 (51.4%), 20.9 (100.0%), 23.1 (17.5%), 24.0 (6.2%), 25.9 (10.2%), 26.7 (30.9%), 28.2 (14.7%), 31.1 (8.8%), 32.1 (7.6%), 33.3 (12.6%), 42.9 (8.0%).

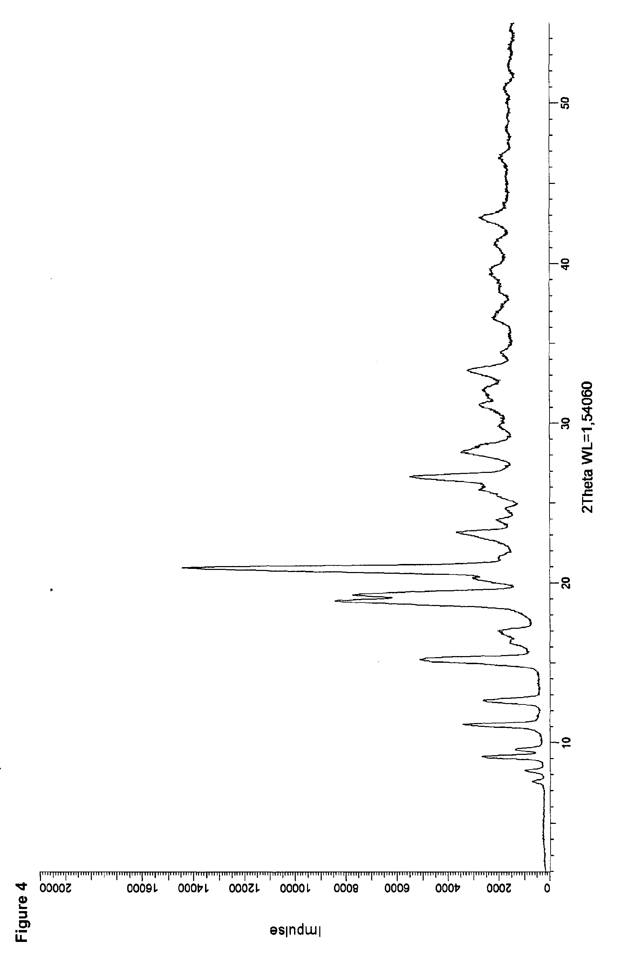

An XRPD pattern of ribociclib free base Form 8 is shown in Figure 4.

Due to the easy access and/or the good solubility in organic solvents ribociclib free base Form 8 can advantageously be used to prepare pharmaceutically acceptable acid addition salts of ribociclib.

Ribociclib free base Form 8 can be obtained by storing crystalline ribociclib free base Form 4 for a time sufficient to convert polymorphic Form 4 into polymorphic Form 8. For example, storing can be conducted at a temperature in the range of 10 to 40°C, preferably at room temperature, such as 23°C. Conversion takes place for example upon storing at 24 to 48 hours, such as for about 36 hours at about 23°C.

The present invention furthermore relates to pharmaceutical compositions comprising crystalline ribociclib free base according to the present invention. The parmaceutical formulation can preferebly be further processed to or be in the form of an oral doasage form, such as a capsule or tablet.

The pharmaceutical composition and/or the oral dosage form of the present invention can be prepared by methods well known to a person skilled in the art such as dry or wet granulation or direct compression.

The pharmaceutical composition can additionally contain one or more pharmaceutically acceptable excipient(s), such as fillers, binders, glidants, disintegrants, lubricants, flow regulating agents and release agents. Suitable excipients are for example disclosed in "Lexikon der Hilfsstoffe fur Pharmazie, Kosmetik und angrenzende Gebiete", published by H.P. Fielder, 4th Edition and "Handbook of Pharmaceutical Excipients", 3rd Edition, published by A.H. Kibbe, American Pharmaceutical Association, Washington, USA, and Pharmaceutical Press, London.

The term filler generally means substances which serve to form the body of the tablet in the case of tablets with small amounts of active agent (e.g. less than 60% by weight). This means that fillers "dilute" the active agent(s) in order to produce an adequate tablet compression mixture. The normal purpose of fillers therefore is to obtain a suitable tablet size. Examples of preferred fillers are lactose, lactose derivatives, starch, starch derivatives, treated starch, chitin, cellulose and derivatives thereof, calcium phosphate, calcium hydrogen phosphate, sucrose, calcium carbonate, magnesium carbonate, magnesium oxide, maltodextrin, calcium sulphate, dextrates, dextrin and/or dextrose, hydrogenated vegetable oil. Fillers can be present in an amount of 0 to 80% by weight, preferably in an amount of 10 to 60% by weight of the total weight of the composition.

A binder is generally a substance which is capable of increasing the strength of the resulting dosage form, especially the resulting tablets. Suitable binders are for example polyvinylpyrrolidone, hydroxypropyl cellulose, hydroxypropyl methylcellulose, methylcellulose, hydroxyethyl cellulose, sugars, dextran, corn starch. Binders can be present in an amount of 0 to 30% by weight, preferably in an amount of 2 to 15% by weight of the total weight of the composition.

Glidants can be used to improve the flowability. Suitable glidants are for example, alkaline earth metal salts of fatty acids, like stearic acid. The glidant can be present for example in an amount of 0 to 2% by weight, preferably in an amount of 0.5 to 1.5% by weight of the total weight of the composition.

Disintegrants are compounds which enhance the ability of the dosage form, preferably the ability of the tablet to break into smaller fragments when in contact with a liquid, preferably water. Suitable disintegrants are for example crosscarmelose sodium, sodium carboxymethyl starch, crosslinked polyvinylpyrrolidone (crosspovidone), sodium carboxymethylglycolate (such as Explotab) and sodium bicarbonate. The disintegrant can be present in an amount of 0 to 20% by weight, preferably in an amount of 1 to 15% by weight of the total weight of the composition.

A suitable flow regulating agent is for example colloidal silica. The flow regulating agent can be present in an amount of 0 to 8% by weight, preferably in an amount of 0.1 to 3% by weight of the total weight of this composition.

A suitable release agent is for example talcum. The release agent can be present in an amount of 0 to 5% by weight, preferably in an amount of 0.5 to 3% by weight of the total weight of the composition.

It is further preferred that the pharmaceutical composition is processed into an oral dosage form. The oral dosage form, preferably a tablet or a capsule, more preferably a tablet, can preferably be coated, preferably film coated.

In the present invention, the following three types of film coatings are possible:
- film coating without affecting the release of the active ingredient,
- gastric juice-resistant film coatings,
- retard film coatings.

Generally, film coatings can be prepared by using film-forming agents such as waxes, cellulose derivatives, poly(meth)acrylate, polyvinylpyrrolidone, polyvinyl acetate phthalate, and/or shellac or natural rubbers such as carrageenan.

It is preferred that the present tablet is coated with a gastric juice-resistant film coating. Alternatively, a capsule comprising a gastric juice-resistant film coating can be used.

The gastric juice-resistant film coating preferably is a film coating being stable in the pH range of about 0.7 to 3.0, which is supposed to be the pH-value of human gastric juice found in the stomach. However, in an environment with a pH value of 5 to 9, which is supposed to be present in the (small) intestine of the human body, the gastric juice-resistant film coating preferably dissolves and the drug can be released.

The gastric juice-resistant film coating (often also referred to as enteric coating) can comprise film-forming agents, for example fats, fatty acids, waxes, alginates, shellac, polyvinyl acetate phthalate, cellulose derivatives such as carboxy methyl ethyl cellulose, cellulose acetate succinate, cellulose acetate phthalate, hydroxypropyl methyl cellulose phthalate, hydroxypropyl methyl cellulose acetate succinate, cellulose acetate trimellitate, and meth(acrylic)acid copolymers such as methyl acrylate-methacrylic acid copolymers, methyl methacrylate-methacrylic acid copolymers, Eudragits (for example Eudragit^{®} L30D, Eudragit^{®} L, Eudragit^{®} S).

The coating is preferably free of active ingredient. It is further preferred that the thickness of the coating is usually 10 µm to 2 mm, preferably from 50 to 500 µm.

The preferred coating may comprise a film-forming agent and one or more of the following: lubricant, surfactant, glidant, pigment and water.

The preferred coating according to an embodiment of the present invention can comprise, along with the film-forming agent, e.g. stearic acid as lubricant for plasticizing and dissolving the polymer, sodium lauryl sulfate as a surfactant for wetting and dispersing, talc as glidant, iron oxide yellow and/or titanium oxide as pigment(s) and optionally purified water.

In a preferred embodiment the pharmaceutical composition can be administered one to three times a day, preferably once or twice a day, more preferably once a day.

The present invention furthermore relates to crystalline ribociclib free base as described above for use in the treatment of systemic diseases, autoimmune diseases or inflammatory diseases, preferably for the use in the treatment of multiple sclerosis, rheumatoid arthritis or psoriasis.

The invention will now be further explained by the following examples, which are not intended to be limiting.

### Experimental part

### Analytical Methods

### X-Ray Powder Diffraction (XRPD)

The sample was analyzed on a D8 Advance X-ray powder diffractometer (Bruker-AXS, Karlsruhe, Germany). The sample holder was rotated in a plane parallel to its surface at 20 rpm during the measurement. Further conditions for the measurements are summarized in the table below. The raw data were analyzed with the program EVA (Bruker-AXS, Germany). The samples were layered onto a silicon specimen holder.

| | standard measurement |
|---|---|
| Radiation | Cu K_{α1/2} (λ=1.5406 Å) |
| Source | 34 kV / 40 mA |
| Detector | Vantec-1 (electronic window: 3°) |
| Kβ filter | Ni (diffracted beam) |
| measuring circle diameter | 435 mm |
| detector window slit | 12 mm |
| anti-scatter (diffracted beam) | 8 mm |
| divergence slit | v6 (variable) |
| soller slit (incident/diffracted beam) | 2.5° |
| 2θ range / ° | 2° ≤ 2θ ≤ 55° |
| step size / ° | 0.016° |
| step time | 0.2s |

### Differential Scanning Calorimetry (DSC)

| | |
|---|---|
| Instrument: | Mettler-Toledo DSC 822E (Mettler-Toledo GmbH, Gießen, Germany) |
| Aluminium crucible: | 40 µL (with perforated lid) |
| Lid: | perforated |
| Temperature range: | 30°C to 350°C |
| Heating rate: | 10°C/ min |
| Nitrogen flush: | 50 mL / min |
| Software: | STARe Version 11.0 |
| Interpretation: | Endothermic mode |

### Dynamic Vapor Sorption - Hygroscopicity

Vapour sorption experiments were performed in the instrument SPSx-1µ (Projekt Messtechnik, Ulm, Germany) at temperature of 25°C and the humidity cycles as shown below. Humidity cycle conditions:

| Cycle | rel. humidity (% RH) | | Number of steps | Time (h) | Comments |
|---|---|---|---|---|---|
| no. | start value | end value | | | |
| 1 | 40 | 0 | 4 | | |
| 2 | 5 | 65 | 6 | | |
| 3 | 75 | 75 | 1 | 24 h | To investigate the absorption of water at the upper humidity level during stress conditions |
| 4 | 85 | 95 | 1 | | |
| 5 | 90 | 0 | 9 | | |
| 6 | 5 | 35 | 3 | | |

### Solubility in aqueous solvents

Approx. 340 mg (exactly weighed) test substance was weighed into a glass vial, followed by addition of 2.5 ml solvent. A stirring bar was added, the vial was fixed in a block heater at 37°C and the suspension was stirred with approx. 250 rpm. After 15 min and 1 h, samples were withdrawn, filtered through a 0.2 µm disposable filter, 50 µl of the clear filtrate were diluted with 950 µl DMSO and 2 µl thereof were analyzed by HPLC/UV.

### Determination of residual solvents by Headspace GC

### Headspace sampler

| | |
|---|---|
| instrument: | Agilent G 1888 |
| oven: | 70 °C |
| loop: | 100 °C |
| transfer line: | 120°C |
| vial equilibration: | 15 min. |
| pressure: | 0.2 min. |
| loop fill: | 0.2 min. |
| inject time: | 0.5 min. |

### GC-MS

| | | |
|---|---|---|
| instruments | | |
| GC: | Agilent 7890A | |
| MSD: | Agilent 5975C | |
| method: | HS EP 35.M | |
| column: | Agilent DB-624 30m x 250 µm x 1.4 µm | |
| temperature program : | 35 °C, 5 min isothermal | |
| | 35-190°C, 5°C / min | |
| | 190°C, 9.4 min isothermal | |
| run time : | 45.4 min | |
| carrier gas : | helium | flow :12.9 ml / min |
| pressure : | 12.4 psi | |
| injection : | split | split ratio : 10 :1 |
| temperature : | inlet: | 220 °C |
| | GC-MS interface : | 280 °C |
| | MS source : | 230°C |
| | MS quad. : | 150 °C |
| MSD pressure : | 9.8 x 10⁻⁶ Torr | |
| tune file : | atune.u | |
| emission : | 34.6 µA | |
| electron energy : | 69.9 eV | |
| repeller : | 27.8 V | |
| ion focus : | 90.2 V | |
| entrance lence : | 25.5 V | |
| EM volts : | 1129.4 V | |
| solvent delay: | 2.5 min | |
| scan : | 35 -500 m/z | |

Ribociclib (RBC) free base was synthesized as described in WO 2012/064805 the whole content of which is incorporated herein by reference.

### Inventive Example 1 Ribociclib free base Form 1

**1a** - 0.3 g (0.7 mmol) amorphous RBC free base as obtained in Reference Example 10 was slurried in 5 mL acetone at RT for 24h. The mixture was filtrated and the obtained solid was dried at 50°C/15mbar for 20h to obtain 0.22 g (73.3%) RBC Form 1 as a white solid.

**1b** - 5 g (11.5 mmol) RBC free base Form 6 as obtained in Inventive Example 5 was slurried in 85 mL acetone at RT for 24h. The mixture was filtrated and the obtained solid was dried at 50°C/15mbar for 20h to obtain 4.67 g (93.4%) RBC Form 1 as a white solid.

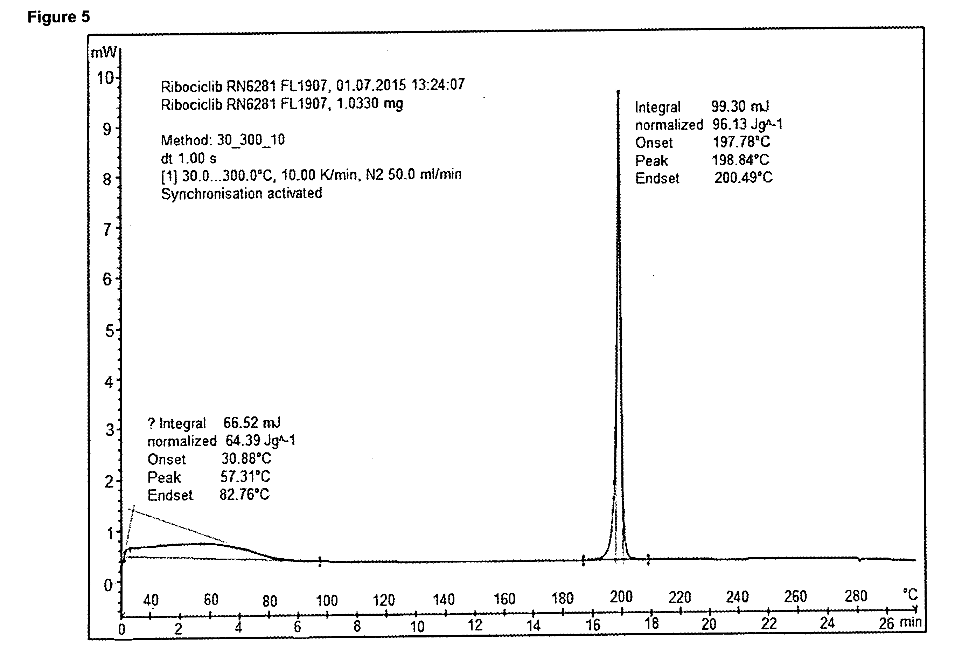

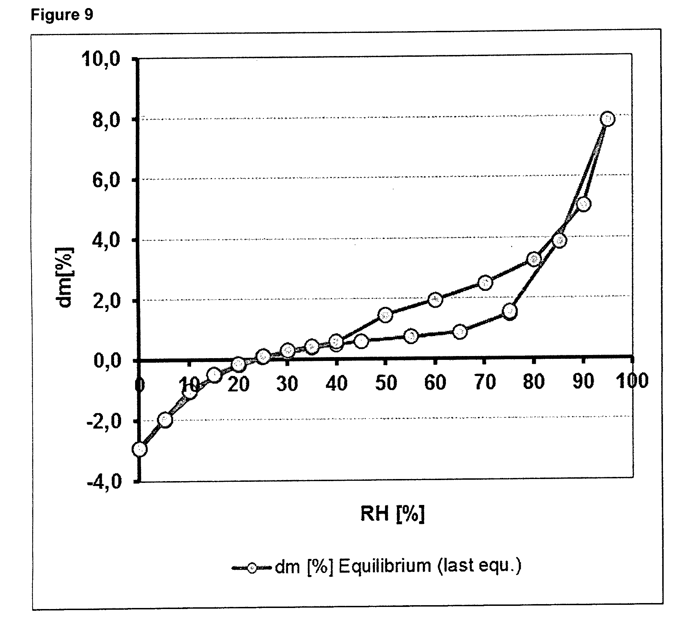

The XRPD pattern and the DSC thermogram of RBC free base Form 1 are shown in Figures 1 and 5. The results of the dynamic vapor sorption test are shown in Figure 9.

### Reference Example 2 Ribociclib free base Form 3

0.5 g (1.2 mmol) RBC free base was dissolved in 5 mL dichloromethane and stirred for 1h at RT. The solution was evaporated to dryness to obtain 0.5 g (100%) RBC Form 3 as a white solid.

### Reference Example 3 Ribociclib free base Form 4

**3a -** 3.0 g (6.9 mmol) RBC free base was slurried in 50 mL methanol at RT for 24h. The mixture was filtrated and the obtained solid was dried at 50°C/15mbar for 90h to obtain 2.48 g (82.7%) RBC Form 4 as a white solid.

**3b -** 0.3 g (0.7 mmol) RBC free base Form 1 as obtained in Inventive Example 1 was slurried in 5 mL methanol at RT for 24h. The mixture was filtrated and the obtained solid was dried at 50°C/15mbar for 20h to obtain 0.20 g (66.7%) RBC Form 4 as a white solid.

**3c -** 0.3 g (0.7 mmol) amorphous RBC free base as obtained in Reference Example 10 was slurried in 5 mL methanol at RT for 24h. The mixture was filtrated and the obtained solid was dried at 50°C/15mbar for 20h to obtain 0.18 g (60.0%) RBC Form 4 as a white solid.

### Reference Example 4 Ribociclib free base Form 5

**4a -** 0.3 g (0.7 mmol) RBC free base was slurried in 5 mL ethanol at RT for 24h. The mixture was filtrated and the obtained solid was dried at 50°C/15mbar for 20h to obtain 0.21 g (70%) RBC Form 5 as a white solid.

**4b -** 0.3 g (0.7 mmol) RBC free base Form 1 as obtained in Inventive Example 1 was slurried in 5 mL ethanol at RT for 24h. The mixture was filtrated and the obtained solid was dried at 50°C/15mbar for 20h to obtain 0.22 g (73.3%) RBC Form 5 as a white solid.

### Inventive Example 5 Ribociclib free base Form 6

**5a -** 0.3 g (0.7 mmol) RBC free base was slurried in 5 mL water at RT for 24h. The mixture was filtrated and the obtained solid was dried at 50°C/15mbar for 20h to obtain 0.23 g (76.7%) RBC Form 6 as a white solid.

**5b -** 5 g (11.5 mmol) RBC free base was dissolved in 12 mL 6N HCl at RT. The water phase was diluted with 9 mL water and the pH was adjusted to ∼12 by the slow addition of 50% aqueous sodium hydroxide at 15°C. The mixture was stirred for 6h at RT. The obtained precipitate was filtrated, washed four times with 20 mL water and dried at 50°C/15mbar for 20h to obtain 5.0 g (100%) RBC Form 6 as a white solid.

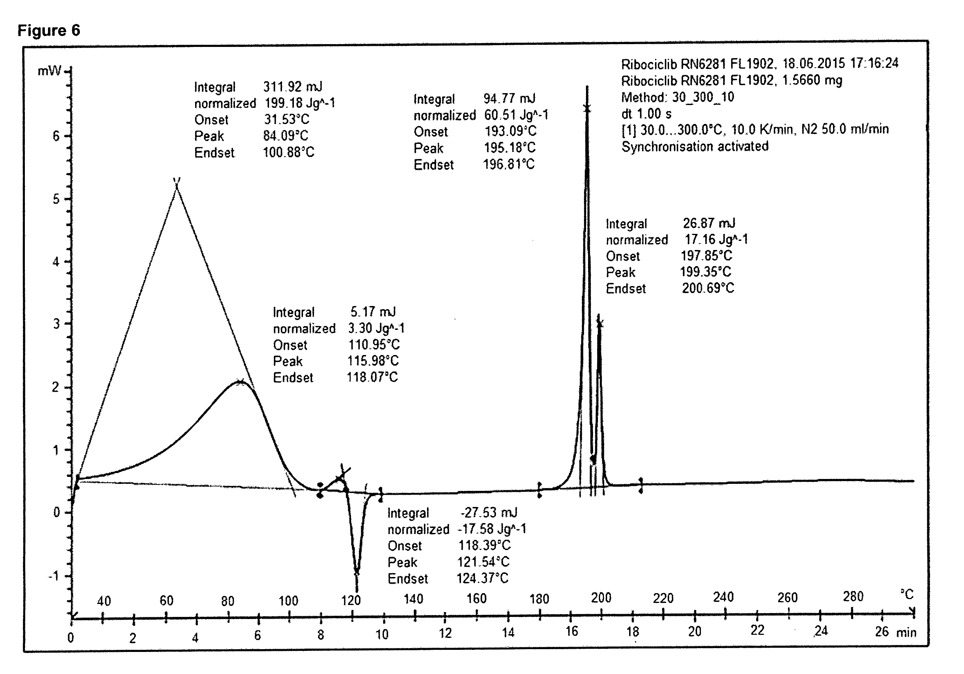

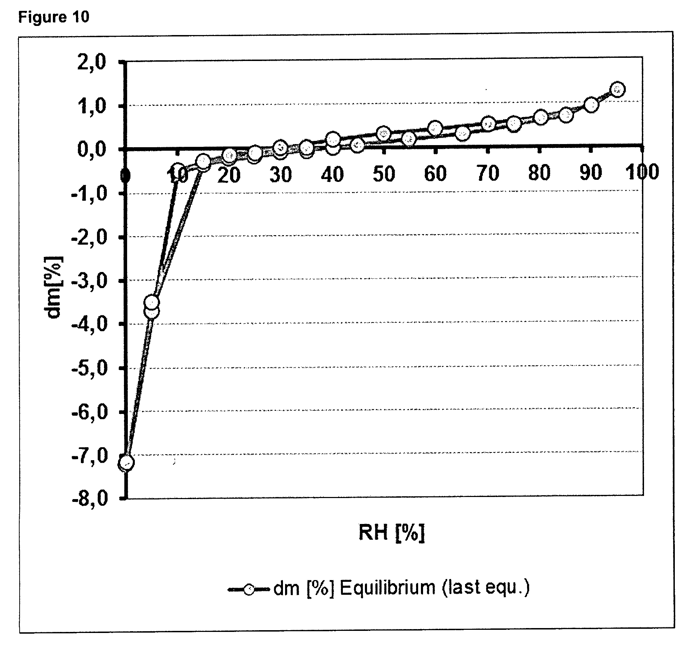

The XRPD pattern and the DSC thermogram of RBC free base Form 6 are shown in Figures 2 and 6. The results of the dynamic vapor sorption test are shown in Figure 10.

### Inventive Example 6 Ribocicilb free base Form 7

**6a -** 0.3 g (0.7 mmol) RBC free base was slurried in 5 mL diisopropylether at RT for 24h. The mixture was filtrated and the obtained solid was dried at 50°C/15mbar for 20h to obtain 0.28 g (93.3%) RBC Form 7 as a white solid.

**6b -** 0.3 g (0.7 mmol) RBC free base was slurried in 5 mL tert-butylmethylether at RT for 24h. The mixture was filtrated and the obtained solid was dried at 50°C/15mbar for 20h to obtain 0.28 g (93.3%) RBC Form 7 as a white solid.

**6c -** 0.3 g (0.7 mmol) RBC free base was dissolved in 66 mL ethylacetate at 65°C and stirred at 50°C for 24h. The mixture was cooled slowly to RT and the obtained precipitate was filtrated and dried at 50°C/15mbar for 20h to obtain 0.19 g (63.3%) RBC Form 7 as a white solid.

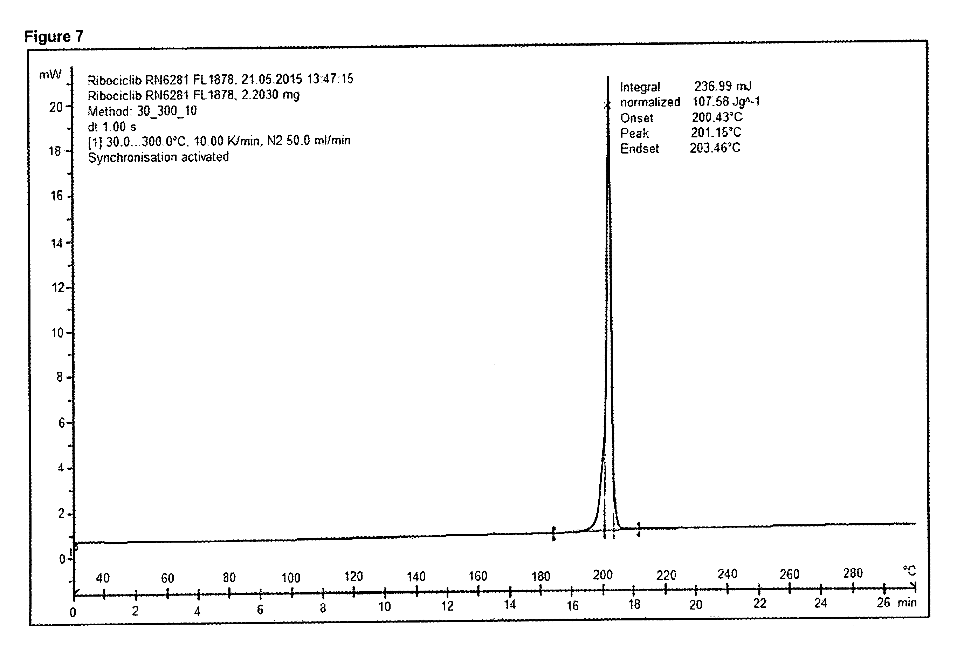

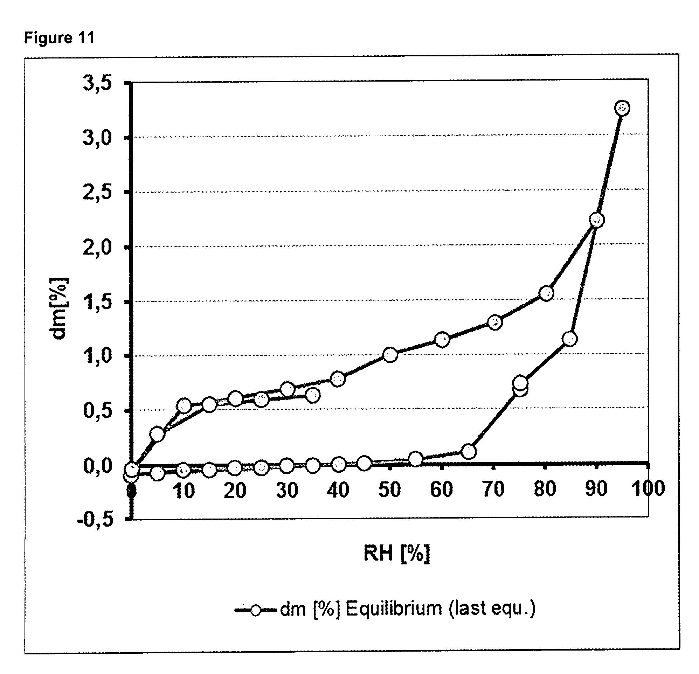

The XRPD pattern and the DSC thermogram of RBC free base Form 7 are shown in Figures 3 and 7. The results of the dynamic vapor sorption test are shown in Figure 11.

### Inventive Example 7 Ribociclib free base Form 8

The RBC free base Form 4 as obtained in Reference Example 3 was stored at RT for 36h to obtain RBC form 8.

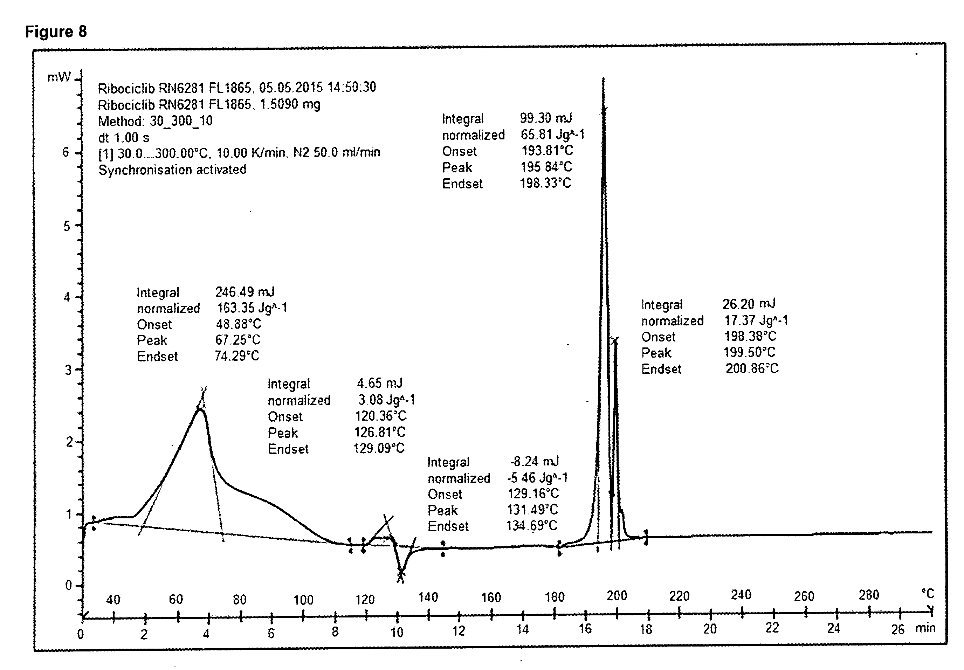

The XRPD pattern and the DSC thermogram of RBC free base Form 8 are shown in Figures 4 and 8.

### Reference Example 8 Ribociclib free base Form 9

0.3 g (0.7 mmol) RBC free base was dissolved in 6 mL dioxane at 80°C and cooled slowly to RT. The obtained precipitate was filtrated and the dried at 50°C/15mbar for 20h to obtain 0.28 g (93.3%) RBC Form 9 as a white solid.

### Reference Example 9 Ribociclib free base Form 10

0.3 g (0.7 mmol) RBC free base was dissolved in 8 mL THF at 65°C and stirred at 50°C for 24h. The mixture was cooled slowly to RT and the obtained precipitate was filtrated and dried at 50°C/15mbar for 20h to obtain 0.22 g (73.3%) RBC Form 10 as a white solid.

### Reference Example 10 amorphous Ribociclib free base

0.5 g (1.2 mmol) RBC free base were pulverized in a "Fritsch pulverisette" for 1h at 400 rpm with 10 balls each having a diameter of 10 mm to obtain amorphous RBC free base.

### Reference Example 11 based on example 3.2 WO2012/064805

0.6 g (1.1 mmol) 4-[6-(7-cyclopentyl-6-dimethylcarbamoyl-7H-pyrrolo[2,3-d]pyrimidin-2-ylamino)-pyridin-3-yl]-piperazine-1-carboxylic acid tert-butyl ester was suspended in 3.4 mL toluene at RT. 1.15 mL 6N HCl was added dropwise maintaining a batch temperature of <15°C. The resulting two-phase solution was stirred for 30 minutes at RT. The phases were separated and the pH of the water phase was adjusted to ∼12 by the slow addition of 50% aqueous sodium hydrogen at 15°C. The mixture was stirred for 3h at RT. The obtained precipitate was filtrated, washed four times with 20 mL water and dried at 50°C/15mbar for 20h to obtain 0.4 g (82.0%) RBC Form 11 as a white solid.

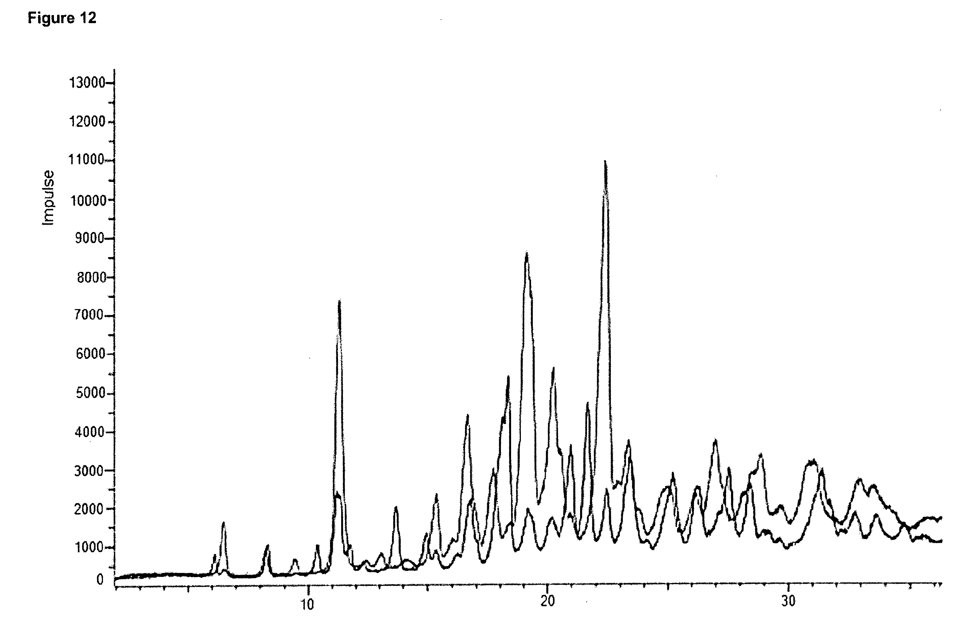

The XRPD pattern of the resulting Form 11 and the XRPD pattern of RBC free base Form 6 (upper line, compare also Figure 2) are shown in Figure 12.

### Example 12 Purity (residual solvent content) and stability of the solid crystalline forms

| | Residual solvents (in ppm) (determined by NMR/HS-GC) | Stability of solid form (in DVS) | Long term stability at 40°C/75%r.h. (4 weeks) | Long term stability at 40°C/75%r.h. (12 weeks) |
|---|---|---|---|---|
| Form 1 | Acetone 83ppm (HS-GC) | stable | stable | stable |
| Form 3 | Dichloromethane 91623ppm (NMR) | N.D. | N.D. | N.D. |
| Form 4 | Methanol 6350ppm (NMR) | Unstable at RT and ambient conditions | N.D. | N.D. |
| Form 5 | Ethanol 44074ppm (NMR) | N.D. | N.D. | N.D. |
| Form 6 | Water | stable | stable | stable |
| Form 7 | Ethylacetate 2700ppm (NMR) | stable | stable | stable |
| Form 8 | Methanol no detected (HS-GC) | N.D. | stable | stable |
| Form 9 | Dioxane 129126ppm (NMR) | N.D. | N.D. | N.D. |
| Form 10 | Tetrahydrofurane N.D. (NMR) | N.D. | N.D. | N.D. |
| Form 11 | Toluene 1159ppm (NMR) | N.D. | N.D. | N.D. |

### Example 13 Solubility in aqueous solvents

| | Solubility [mg/ml] | | | |
|---|---|---|---|---|
| experiment | 0.01 N HCl ∼pH2.2 | 20mM NaAc pH4.5 | 50mM KH₂PO₄ pH6.8 | 50mM KH₂PO₄ pH6.5 + 2%TPGS |
| RBC free base form 1 | | | | |
| 15 min | 3.8 | 14.2 | 1.5 | 3.6 |
| 1 h | 3.4 | 14.2 | 1.6 | 1.5 |
| RBC free base form 6 | | | | |
| 15 min | 0.4 | 12.2 | 1.2 | 2.8 |
| 1 h | 0.2 | 9.7 | 0.5 | 2.4 |
| RBC free base form 7 | | | | |
| 15 min | 3.1 | 11.3 | 0.5 | 0.8 |
| 1 h | 3.0 | 11.5 | 1.1 | 0.8 |
| RBC free base form 8 | | | | |
| 15 min | 4.0 | - | - | 3.9 |
| 1 h | 1.0 | - | - | 0.9 |

## Claims

1. Crystalline ribociclib free base, having a residual solvent content within pharmaceutically acceptable limits.

2. Crystalline ribociclib free base Form 1 according to claim 1, having characteristic X-ray powder diffraction peaks at 12.5, 12.6, 13.3, 14.8 and 18.3 degrees 2θ (± 0.2 degrees 2θ).

3. Crystalline ribociclib free base Form 1 according to claim 2, having one or more further characteristic X-ray powder diffraction peak(s) at 13.6, 18.1, 21.8, 22.9 and/or 26.9 degrees 2θ (± 0.2 degrees 2θ).

4. Crystalline ribociclib free base Form 6 according to claim 1, having characteristic X-ray powder diffraction peaks at 6.4, 11.3, 16.6, 19.1 and 22.4 degrees 2θ (± 0.2 degrees 2θ).

5. Crystalline ribociclib free base Form 6 according to claim 4, having one or more further characteristic X-ray powder diffraction peak(s) at 8.2, 15.3, 17.7, 20.2 and/or 28.8 degrees 2θ (± 0.2 degrees 2θ).

6. Crystalline ribociclib free base Form 7 according to claim 1, having characteristic X-ray powder diffraction peaks at 5.3, 11.0, 13.9, 14.7 and 15.9 degrees 2θ (± 0.2 degrees 2θ).

7. Crystalline ribociclib free base Form 7 according to claim 6, having one or more further characteristic X-ray powder diffraction peak(s) at 10.7, 13.0, 20.8, 21.1 and/or 23.2 degrees 2θ (± 0.2 degrees 2θ).

8. Crystalline ribociclib free base Form 8 according to claim 1, having characteristic X-ray powder diffraction peaks at 9.1, 11.1, 15.2, 18.9 and 20.9 degrees 2θ (± 0.2 degrees 2θ).

9. Crystalline ribociclib free base Form 8 according to claim 8, having one or more further characteristic X-ray powder diffraction peak(s) at 7.6, 8.2, 12.7, 26.7 and/or 28.2 degrees 2θ (± 0.2 degrees 2θ).

10. Method of preparing crystalline ribociclib free base Form 1 according to claim 2 or 3, which comprises slurrying amorphous ribociclib free base or crystalline ribociclib free base Form 6 in acetone.

11. Method of preparing crystalline ribociclib free base Form 6 according to claim 4 or 5, which comprises slurrying ribociclib free base of any form being different to Form 6 in water or dissolving ribociclib free base in an aqueous solvent at low pH and precipitating crystalline ribociclib free base from the obtained solution by increasing the pH.

12. Method of preparing crystalline ribociclib free base Form 7 according to claim 6 or 7, which comprises dissolving ribociclib free base in ethylacetate and precipitating crystalline ribociclib free base from the obtained solution.

13. Method of preparing crystalline ribociclib free base Form 8 according to claim 8 or 9, which comprises storing crystalline ribociclib free base Form 4.

14. Pharmaceutical composition comprising ribociclib free base according to any one of claims 1 to 9, preferably being a solid oral dosage form.

15. Crystalline ribociclib free base according to any one of claims 1 to 9 for use in the treatment of systemic diseases, autoimmune diseases or inflammatory diseases, preferably for the use in the treatment of multiple sclerosis, rheumatoid arthritis or psoriasis.
